# FASCICULE DE BREVET EUROPEEN

(11) **EP 0 909 155 B1**
(45) Date de publication et mention de la délivrance du brevet: **26.11.2003**
(21) Numéro de dépôt: 97927234.1
(22) Date de dépôt: 04.06.1997
(51) Int. Cl.: A61J 1/00, A61M 5/28, A61M 5/315, A61M 5/00

(54) **PROCEDE POUR CONSTITUER UNE PREPARATION INJECTABLE ET DISPOSITIF POUR LA MISE EN OEUVRE DE CE PROCEDE**
VERFAHREN ZUM ANFERTIGEN EINES INJIZIERBAREN PRÄPARATES UND VORRICHTUNG ZUM DURCHFÜHREN DES VERFAHRENS
METHOD FOR PREPARING AN INJECTABLE PREPARATION AND DEVICE FOR IMPLEMENTING SAME

(30) Priorité: 04.06.1996 FR 9606886
(43) Date de publication de la demande: 21.04.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHERIF CHEIKH, Roland, F-92130 Issy les Moulineaux (FR)
(74) Mandataire: Moncheny, Michel
(86) Numéro de dépôt international: FR9700989
(87) Numéro de publication internationale: WO97046202

(56) Documents cités:
- EP-A- 0 161 797
- EP-A- 0 499 481
- EP-A- 0 520 618
- WO-A-94/01150
- WO-A-94/06690
- WO-A-96/29113
- US-A- 3 654 926
- US-A- 3 810 469
- US-A- 4 243 080
- US-A- 5 334 162

## Description

La présente invention a pour objet un procédé pour constituer une préparation pharmaceutique injectable et un dispositif pour la mise en oeuvre de ce procédé.

On sait que les formes injectables sont immédiatement biodisponibles et constituent un mode possible d'administration passive pour le malade et un traitement idéal dans les cas d'urgence.

Une autre raison importante au développement des formes parentérales est l'utilisation de principes actifs (PA) qui sont dégradés et/ou non absorbés significativement par la voie orale.

Parmi tous ces P.A. qui, pour diverses raisons, nécessitent une forme injectable, nombreux sont ceux qui sont instables en milieu aqueux, que ce soit en solution, en suspension ou en dispersion.

Pour éviter l'hydrolyse et l'ensemble des problèmes physico-chimiques liés à une présentation liquide (précipitation, agrégation, adsorption, cristallisation,...), on utilise souvent une présentation dans laquelle le P.A. est conservé sous une forme solide, séchée ou lyophilisée.

La préparation de la forme liquide nécessaire à l'injection a alors lieu extemporanément, juste avant l'injection.

Cette préparation consiste à hydrater la forme solide avec le milieu liquide de solubilisation ou de suspension du P.A.

Classiquement, cette opération est réalisée dans un flacon scellé qui contient la forme solide. Le liquide est introduit dans le flacon grâce à une seringue dont l'aiguille peut transpercer un bouchon-septum.

La forme liquide est ensuite récupérée dans la seringue pour pouvoir être injectée.

Le temps nécessaire à cette opération délicate, et les risques de contamination qu'elle comporte, ont conduit les spécialistes de technologie médicale à imaginer des dispositifs pour rendre plus sûre et plus simple la préparation extemporanée, et utiliser le moins possible d'éléments.

Dans ce sens, on connaît par les brevets EP-A-0 664 136, DAIKYO SEIKO, EP-0 599 649 PHARMACIA, WO-95 11051, une seringue bicompartimentale ou "by-pass", qui associe dans une même seringue le milieu liquide et la forme solide qui sera directement réhydratée dans la seringue avant injection.

De même, certains fabricants proposent des dispositifs qui associent le flacon à la seringue et contrôlent la bonne exécution de la préparation (brevets français DEBIOTECH 2 705 898, 2 715 311, 2 717 086).

Certains défauts des systèmes traditionnels ne sont, toutefois, pas résolus par ces nouveaux dispositifs, qui posent des problèmes de perte de préparation dans leurs volumes morts, qui ne rendent pas la réhydratation automatique et statique mais la maintiennent manuelle et dynamique, c'est-à-dire que le flot de liquide et les transferts après hydratation déplacent la forme solide et notamment le P.A. La forme injectable n'a, alors, pas nécessairement la même répartition ou homogénéité que la forme sèche. Ceci constitue un problème notamment dans le cas des suspensions.

Le fait que la préparation soit dynamique et réalisée manuellement peut conduire à des différences importantes selon l'opérateur, la vitesse avec laquelle il agit, la façon dont il charge le liquide et dont il contrôle l'évacuation de l'air. Enfin, la force avec laquelle le P.A. solide est hydraté est susceptible d'entraîner une émulsion de microbulles d'air plus ou moins importante.

Le temps laissé à la solubilisation ou à la suspension et l'agitation du milieu liquide conditionnent l'homogénéité de la préparation.

Dans le cas des suspensions, une mauvaise homogénéité ou un début de sédimentation peut entraîner des problèmes de dose et d'administration.

Un procédé/un dispositif selon le préambule de la revendication 1/10 est connu du document WO-A-94/01150.

L'invention a pour but de proposer un procédé remédiant à ces divers inconvénients.

Conformément à l'invention, le procédé pour constituer une préparation injectable présente les étapes selon la revendication 1.

Le procédé de préparation et de conditionnement sous vide de l'invention évite à la fois les problèmes ci-dessus (volume mort, activation manuelle, injectabilité) et les problèmes de formulation liquide injectée (homogénéité, dégazage).

Suivant un mode de réalisation du procédé de l'invention, on ajoute à la forme sèche une couche d'excipient qu'on utilise à la suite de la préparation injectable comme un piston liquide pour pousser les autres couches et réduire les pertes de principe actif lors de l'injection.

Suivant une autre forme de réalisation du procédé conforme à l'invention, la forme sèche est conditionnée dans une seringue fixée à un dispositif automatique de réhydratation ; pour préparer la forme sèche , on congèle un liquide contenant le principe actif, on ajoute en surface du liquide congelé une quantité déterminée de solution d'excipient, on congèle cette solution d'excipient, on lyophilise l'ensemble ; on obtient ainsi, entre le piston de la seringue et le solide de principe actif sous vide, un volume de lyophilisat ne contenant que l'excipient qui, après réhydratation automatique et déplacement du piston pour vider la seringue, occupe en fin d'injection un volume mort au fond de la seringue et de l'aiguille d'injection.

Ce procédé de préparation et de conditionnement conduit à une réhydratation automatique : il suffit à l'utilisateur d'activer le dispositif pour que le liquide reconditionne la forme solide dans l'état où elle se trouvait avant séchage ou lyophilisation. L'activation du dispositif consiste à mettre en contact le volume liquide et le volume solide sous vide. Après activation, la préparation extemporanée est automatique, c'est-à-dire que les éléments du dispositif se déplacent seuls sous l'action du liquide qui est attiré par aspiration par le vide dans lequel se trouve la formulation solide.

Cette propriété du conditionnement sous vide est indépendante de l'opérateur et l'hydratation conduit à un retour immédiat à la situation de la forme liquide avant séchage ou lyophilisation.

La forme solide et le principe actif restent statiques au cours de cette hydratation, c'est-à-dire non déplacés par le liquide.

Cette préparation immédiate est donc directement injectable sans qu'il soit nécessaire de l'agiter, de la transférer ou de chasser l'air avant injection.

Ce procédé de préparation et de conditionnement peut utiliser certains dispositifs ou contenants actuellement disponibles, à condition qu'ils assurent le maintien sous vide de la forme sous vide jusqu'à réhydratation. Le ou les éléments du dispositif doivent permettre cette réhydratation en évitant le contact avec l'air ambiant.

Cette particularité conduit aussi à certains dispositifs ou éléments spécifiques pour ce procédé de préparation et conditionnement. Ces dispositifs et éléments seront décrits plus loin.

Les techniques de conditionnement sous vide de la forme solide dans le dispositif et l'emballage sont dérivées de techniques existantes (tube de prélèvement sanguin, emballage sous film plastique...). Ce conditionnement sous vide de la forme solide et du P.A. est, de plus, susceptible de remplacer l'inertage (azote) et d'améliorer la stabilité de la préparation en particulier à la température (isolement thermique) et la compatibilité au contenant (isolement de contact).

Les avantages précédemment mentionnés du procédé et des dispositifs de l'invention, qui seront expliqués plus loin, sont particulièrement importants pour certaines préparations :
- Pour une préparation solide facilement solubilisable, l'avantage est d'obtenir immédiatement une préparation liquide sans bulles d'air et dégazée.
- Pour une préparation solide plus difficilement solubilisable, soit par sa viscosité soit par le temps nécessaire à la solution, la formulation sous vide évite l'émulsion d'air dans le liquide, simplifie et accélère la solubilisation.
- Pour une suspension et plus spécialement pour une suspension de microsphères retard (Décapeptyl 3,75 B.I.), la formulation sous vide évite les problèmes de déshomogénéisation et les risques de précipitation, donc l'obturation en diminuant le temps nécessaire à la reconstitution.

La préparation galénique sous vide et le dispositif préchargé permettent de diminuer considérablement le volume mort, et donc les pertes de principe actif.
- Enfin pour une dispersion, et plus particulièrement, pour les formes semi-solides, la très forte viscosité de la forme hydratée rend pratiquement indispensable le recours à un procédé de préparation et de conditionnement sous vide de la forme sèche.

La forme aqueuse non-liquide ou semi-solide obtenue après hydratation sous vide est, de plus, susceptible d'avoir des propriétés de relargage différentes et meilleures que celles d'une forme hydratée à l'air. Le fait de ne pas avoir d'air prisonnier dans la dispersion permet de diminuer le volume pour une même quantité (ce qui améliore le relargage) et évite les ruptures de la structure en dépôt in-situ qui peuvent également modifier le relargage.

Le procédé, le conditionnement et les dispositifs sont, ici, décrits pour des formes liquides aqueuses. Il va de soi que l'ensemble de l'invention s'applique avec les mêmes avantages aux formes liquides (solution, suspension ou dispersion), reconstituées à partir d'un mélange eau-solvant organique, à partir de solvant organique ou à partir d'autres liquides comme les huiles injectables.

La vitesse du processus de préparation et sa réalisation dans un conditionnement hermétique compensent la viscosité ou les risques d'évaporation de certains liquides.

Le dispositif pour la mise en oeuvre du procédé selon l'invention présente les caractéristiques selon la revendication 10.

Conformément à un mode de réalisation préférentiel, les moyens de conditionnement sous vide de la forme sèche sont une seringue étanche aux gaz, et les moyens de conditionnement de liquide sont un réservoir contenant un piston.

La seringue est préchargée de la forme solide sous vide, dont le conditionnement permet une injection immédiate après hydratation sans agitation, et en évitant le transfert de la solution ou de la suspension à travers une aiguille depuis le réservoir de préparation du liquide vers la seringue.

Un autre avantage des dispositifs conditionnés sous vide est qu'il est ainsi possible de diminuer les volumes des réservoirs du liquide et du solide tout en augmentant la précision du volume injecté.

En effet, l'absence d'air permet de remplir complètement le compartiment contenant le solide. Le volume contenu dans le compartiment liquide peut être exactement calculé pour occuper le volume laissé vide dans la préparation plus les pertes du dispositif. Mais ce volume peut être également en excès, car c'est le volume du vide dans le solide qui fixera exactement la quantité de liquide nécessaire à la réhydratation.

Le dispositif contenant le liquide de réhydratation est avantageusement contenu dans un réservoir étanche, dont le volume peut diminuer sans contrainte au fur et à mesure que le liquide est transvasé vers le réservoir de la forme solide sous vide.

Ceci peut notamment être obtenu facilement avec une carpule ou cartouche ou avec une seringue pré-remplie, dont le piston se déplace avec le mouvement du liquide.

Le réservoir pourra également être constitué d'une poche plastique souple pré-remplie, dont les parois flexibles suivront le déplacement du liquide.

L'élément de connexion du liquide et du vide à l'abri de l'air ambiant pourra être constitué d'un septum, d'un clapet, d'une vanne ou d'un robinet.

Une des caractéristiques du procédé et des dispositifs est de diminuer les volumes morts. Cela est réalisé non seulement en diminuant le volume des éléments de connexion (liquide-vide) ou d'injection (aiguille-seringue) mais aussi grâce au processus statique de réhydratation qui permet d'occuper les volumes morts avec du liquide sans principe actif, et donc sans perte d'injection.

Ainsi, l'élément connecteur et/ou l'aiguille pourront être chargés de liquide sans principe actif pour éliminer les pertes.

De plus, il est possible, grâce au même processus statique, de prévoir le "piston liquide" sans principe actif mentionné précédemment, qui occupera les volumes morts de la seringue d'injection et de l'aiguille après administration, permettant ainsi de réduire encore les pertes de principe actif. Ceci est simplement obtenu en ajoutant, après congélation de la formulation liquide contenant le principe actif, un volume calculé d'une solution d'excipient tel que du mannitol, qui sera congelé et lyophilisé en même temps que la formulation. Grâce à la réhydratation statique et rapide, les deux liquides, une fois reformés, ne se mélangeront pratiquement pas et le liquide sans principe actif pourra pousser tout le liquide avec principe actif hors de la seringue et de l'aiguille (comme un "piston liquide"), ce qui évitera les pertes.

Dans tous les cas (solution, suspension ou dispersion), une fois la forme solide séchée ou lyophilisée, si la seringue est fermée du côté de l'injection par l'élément de connexion, l'aiguille ou un septum, on positionne le piston sous vide avec ou sans système de blocage, par exemple, à l'intérieur du lyophilisateur. Si la seringue est ouverte, on peut la conditionner sous vide d'air au moment de l'emballage sous film de plastique.

Même si le conditionnement de la seringue fermée est au préalable réalisé sous vide, on aura avantage à emballer ensuite cette seringue sous vide de façon à ce que l'emballage, et non la seringue, assure l'étanchéité à l'air lors du stockage. Ceci constitue une double sécurité et facilite de plus le contrôle de l'intégrité de l'emballage avant utilisation (ouverture).

Le produit ou forme finale obtenu après hydratation du solide peut se présenter sous l'une des trois formes ci-dessous :

### 1) Solutions

Le principe actif associé, par exemple, au mannitol, est solubilisé dans l'eau pour préparation injectable; la solution est distribuée en volume à l'intérieur des seringues; les seringues sont congelées et lyophilisées selon un procédé traditionnel et le lyophilisat solide est conditionné sous vide avec la seringue préfixée ou non aux autres éléments du dispositif de réhydratation extemporanée.

### 2) Suspensions

Dans le cas, par exemple, des microsphères retard, la dose de microsphères est pesée dans la seringue. On ajoute le volume de liquide de dispersion. On disperse ensuite mécaniquement les microsphères dans le liquide. Préférentiellement, on utilise les ultrasons pour cette dispersion. On congèle, ensuite, rapidement la dispersion, préférentiellement dans l'azote liquide. On obtient alors une dispersion homogène des microsphères dans le liquide congelé. Le liquide contient la trame du lyophilisat, par exemple, le mannitol. On lyophilise et on obtient un solide dans lequel les microsphères sont suspendues par la trame dans l'état idéal de dispersion homogène du liquide.

On conditionne alors sous vide dans la seringue ce solide, associé ou non aux éléments du dispositif automatique de réhydratation extemporanée.

### 3) Dispersion

Dans le cas d'un implant semi-solide, par exemple du semi-solide Autogel BIM 23014 C, le principe actif est pesé à l'intérieur d'une seringue de dosage étanche aux gaz.

Le produit obtenu par la mise en oeuvre du procédé et du dispositif selon l'invention comprend une forme sèche pour administration parentérale et conditionnée sous vide à l'intérieur d'un dispositif d'injection contenant également un volume liquide, prêt à être mélangé par aspiration avec la forme sèche pour reconstituer la préparation injectable.

La forme sèche peut être une forme lyophilisée ou une poudre obtenue après élimination d'un solvant.

D'autres particularités et avantages de l'invention apparaîtront au cours de la description qui va suivre, faite en référence aux dessins annexés qui en illustre un certain nombre de formes de réalisation à titre d'exemples non limitatifs.
La figure 1 est une vue en élévation d'une seringue contenant un principe actif et étanche au gaz.
La figure 2 illustre la mise en compression du principe actif dans la seringue de la figure 1.
La figure 3 illustre la mise sous vide d'air du principe actif de la seringue de la figure 2,
seringue La figure 4 est une vue en élévation longitudinale de la seringue de la figure 3 connectée à un réservoir de liquide par l'intermédiaire d'une vanne, celle-ci étant en position de fermeture,
la figure 5 illustre l'aspiration du liquide dans la seringue contenant le solide après ouverture de la vanne,
La figure 6 représente une phase complémentaire de mélange entre le liquide et le solide par aspiration de ce mélange dans le réservoir initialement rempli de liquide,
La figure 7 illustre une étape de chargement d'une petite seringue à partir de la seringue précédemment remplie et séparée de la seconde seringue,
la figure 8 est une vue de la petite seringue de la figure 7 prête à être connectée à un réservoir de liquide,
La figure 9 est une vue de la seringue de la figure 8 connectée à un réservoir de liquide avec interposition d'une vanne.
La figure 10 est une vue de l'ensemble seringue-réservoir de liquide de la figure 9 emballé sous vide d'air.
Les figures 11 à 14 illustrent les étapes successives de la mise en oeuvre d'un premier mode de réalisation du procédé et du dispositif selon l'invention.
Les figures 15 à 17 sont des vues en élévation analogues aux figures 11 à 14 illustrant la mise en oeuvre d'un second mode de réalisation du dispositif selon l'invention.
Les figures 18 à 21 illustrent la mise en oeuvre d'un troisième mode de réalisation du procédé et du dispositif selon l'invention.
La figure 22 est une vue en élévation longitudinale d'un quatrième mode de réalisation du dispositif selon l'invention.
Les figures 23 à 25 montrent les étapes successives de la constitution et du conditionnement en emballage sous vide d'un cinquième mode de réalisation du dispositif selon l'invention.
Les figures 26 à 29 sont des vues en élévation longitudinale et coupe partielle illustrant la mise en oeuvre d'un sixième mode de réalisation du dispositif selon l'invention.
Les figures 30 à 32 sont des vues en élévation longitudinale illustrant la mise en oeuvre d'un septième mode de réalisation de l'invention.
Les figures 33 à 36 sont des vues illustrant la mise en oeuvre d'un huitième mode de réalisation du dispositif selon l'invention.
Les figures 37, 38 et 39 illustrent les étapes successives de la mise en oeuvre d'un neuvième mode de réalisation du dispositif de seringue selon l'invention.
Les figures 40, 41 et 42 sont des vues en élévation partielle illustrant trois variantes d'exécution possibles de la réalisation des figures 37 à 39.
La figure 43 est une vue en coupe élévation partielle d'un dixième mode de réalisation du dispositif selon l'invention.
La figure 44 est une vue analogue à la figure 40 d'un onzième mode de réalisation du dispositif selon l'invention.
La figure 45 est une vue en coupe longitudinale et élévation partielle d'un douzième mode de réalisation du dispositif de seringue selon l'invention.
La figure 46 est une vue en coupe longitudinale et élévation partielle d'un treizième mode de réalisation du dispositif de conditionnement visé par l'invention.

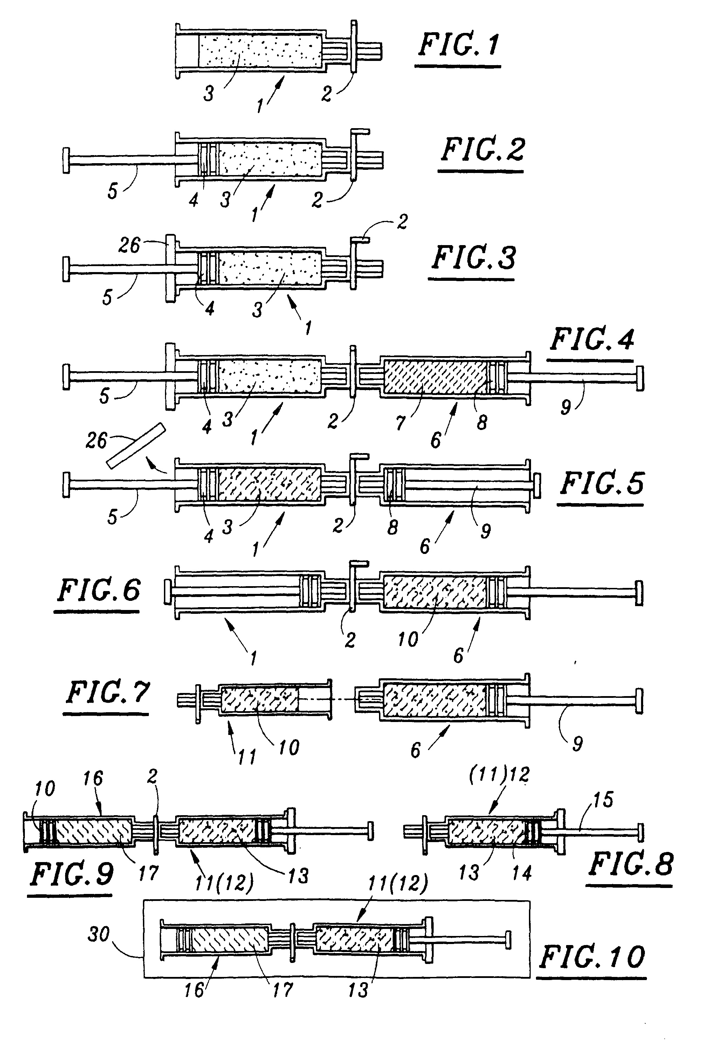

On décrira tout d'abord en référence aux figures 1 à 10, un mode de réalisation du procédé pour constituer une préparation injectable selon l'invention, et du dispositif de réhydratation sous vide pour la mise en oeuvre de ce procédé.

On charge dans une seringue 1, munie d'un robinet ou vanne 2 à la place de son aiguille d'injection, un principe actif 3 pesé et ramené à un volume proche ou égal à celui occupé par la forme semi-solide par pression du piston 4 (figure 2) de la seringue 1 avant ou après mise sous vide d'air. Aux figures 2 et 3, on peut admettre par exemple que le principe actif 3 est comprimé par le piston 3 porté par sa tige 5, avant mise sous vide d'air, opération effectuée au stade de la figure 3. On équipe la tige 5 d'une pièce 26 de retenue du piston 4 prenant appui sur l'extrémité de la seringue 1, et ce en raison du vide dans le volume du principe actif 3.

Le principe actif 3 peut être prétraité pour s'adapter au volume final et/ou pour favoriser l'hydratation suivante. On peut ainsi calculer sa granulométrie par broyage, par spray-drying ou par lyophilisation à une concentration déterminée.

La seringue 1 de dosage contenant le principe actif sous vide 3 est alors connectée (figure 4) par la vanne étanche 2 avec une même seringue 6 contenant un volume 7 de liquide de réhydratation du solide 3, par exemple de l'eau. Ce volume liquide 7 est contenu dans la seringue 6 par le piston 8 et la tige 9 de cette seringue.

Puis la vanne 2 est ouverte (figure 5), de sorte que le liquide 7 passe dans le solide 3 par aspiration sous l'action du vide, plus éventuellement par une action mécanique sur le piston 8. La pièce de retenue 26 peut alors être enlevée.

La préparation se mélange donc d'abord dans la seringue 1, puis est ensuite à nouveau mélangée immédiatement, ou après un temps d'hydratation, par va et vient d'une seringue à l'autre (figure 6). Ce va et vient est obtenu par action mécanique sur le piston 8 et sur le piston 4, par exemple avec un pousse-seringue ou une presse hydraulique.

Une fois homogène, le mélange est immédiatement, ou après un temps de repos, réparti à l'intérieur de petites seringues telles que 11 du dispositif d'injection par dosage volumétrique à partir d'une des deux seringues 1 et 6 de mélange qui sont de grandes dimensions. Si la quantité et la précision du dosage dans chaque seringue 11 ne permet pas une répartition directe à partir de la seringue de préparation du mélange, notamment lorsque la seringue de mélange 6 correspond à un volume important, on utilise alors une seringue intermédiaire de plus faible diamètre pour la répartition.

La seringue 11 peut être par exemple une telle seringue intermédiaire. Le contenu de la grande seringue 6 est ainsi réparti dans plusieurs seringues intermédiaires 11 de plus faible capacité, chacune d'elle étant ensuite dans une étape finale vidée dans plusieurs petites seringues de faible contenance.

Par exemple, à partir d'un lot préparé dans des seringues 6 de 200ml, on peut utiliser 10 seringues de 10ml pour charger des unidoses de 0,2ml dans les seringues finales.

Les seringues finales 11 ou 12 remplies du semi-solide 13 et équipées de leur piston 14 et de leur tige 15 (figure 8) sont ensuite lyophilisées et conditionnées sous vide d'air, puis associées (figure 9) à un dispositif automatique 16 de réhydratation extemporanée. Ce dispositif 16 peut être lui-même une seringue contenant le liquide 17 et raccordé à la seringue 11 ou 12 par une vanne étanche 2.

En définitive, le dispositif ainsi obtenu (figure 9) est conditionné dans un emballage sous vide 18, prêt à l'utilisation pour une injection de la préparation injectable qui sera obtenue par mélange du liquide 17 et du solide 13, par aspiration grâce à l'action du vide dans lequel se trouve le solide 13.

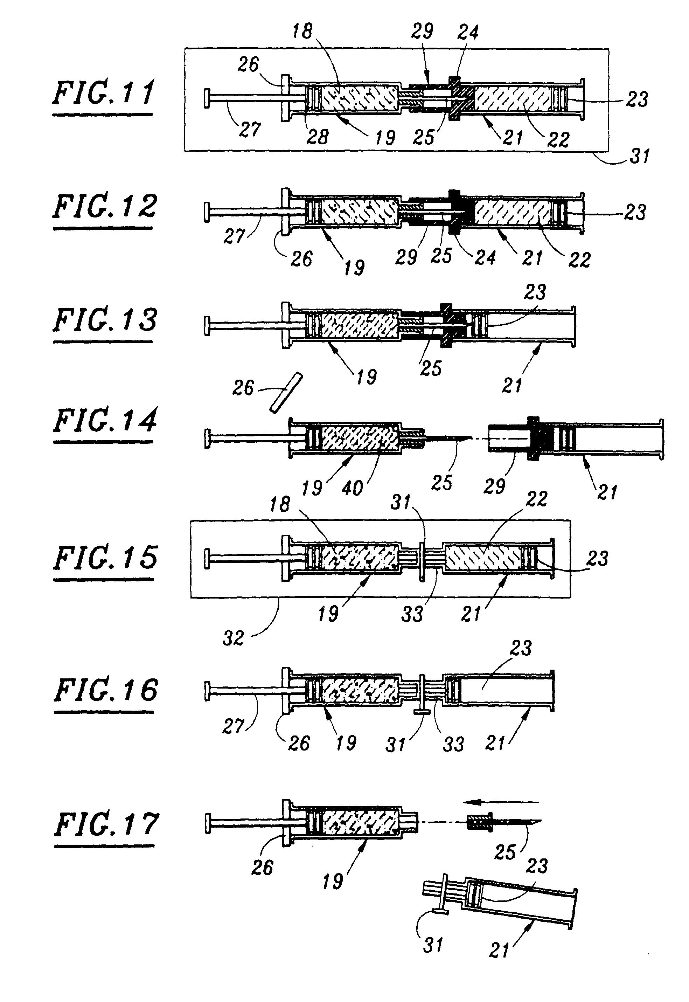

Dans le mode de réalisation des figures 11 à 14, la forme sèche 18 est conditionnée dans une seringue 19 fixée à un dispositif automatique 21 de réhydratation, contenant un volume 22 de liquide, constitué par un réservoir contenant un piston 23. Le lyophilisé ou solide 18 est préchargé sous vide à l'intérieur de la seringue 19 avant connexion de celle-ci avec le réservoir de liquide 21. Les moyens de connexion entre le réservoir 21 et la seringue 19 sont, dans l'exemple représenté, constitués par un raccord 28 comportant un septum 24, dans lequel est enfoncée l'extrémité d'une aiguille d'injection 25 de la seringue 19. Cette dernière est en outre munie d'une pièce 26 maintenant la tige 27 et son piston 28 dans la position adéquate, compte tenu du vide sous lequel est placé le solide 18. L'ensemble du dispositif est conditionné sous vide dans un emballage souple 31.

Pour obtenir la préparation injectable à partir du dispositif de la figure 11, on enlève d'abord l'emballage 31, puis on pousse la seringue 19, par sa tige 27, de façon à enfoncer l'aiguille 25 dans le septum 24 (figures 12, 13). Lorsque l'extrémité de l'aiguille 25 pénètre dans le volume de liquide 22, celui-ci est aspiré par le vide régnant dans le solide 18, avec lequel il se mélange sans modification du volume occupé par le solide 18, tandis que le piston 23 coulisse en direction de la seringue 19. Après quoi l'utilisateur enlève la pièce 26, le réservoir 21 et le capuchon-septum 29 (figure 14), et la seringue 19 est prête à l'utilisation pour l'injection de la préparation qu'elle contient.

Dans l'exemple illustré aux figures 15 à 17, le lyophilisé ou solide 18 préchargé sous vide dans la seringue 19 est connecté avec le réservoir de liquide 21 (ici une carpule comme dans l'exemple des figures 11 à 14) par l'intermédiaire d'une vanne 31 par exemple du type quart de tour. La seringue 19 est conditionnée sous vide dans un emballage souple 32 et fixée à un connecteur 33 de liaison avec le réservoir 21 de liquide, le connecteur 33 étant équipé de la vanne 31.

La préparation extemporanée de réhydratation consiste alors à ouvrir la vanne 31 pour que le liquide 22 passe automatiquement, par aspiration, de la carpule 21 à la seringue 19 (figure 16) tandis que le piston 23 de la carpule 21 parcourt sa course vers la seringue 19. On déconnecte ensuite la carpule 21 et le robinet 31 (figure 17) de la seringue 19 pour fixer sur celle-ci l'aiguille d'injection 25, le mélange obtenu dans la seringue 19 étant alors prêt pour être injecté.

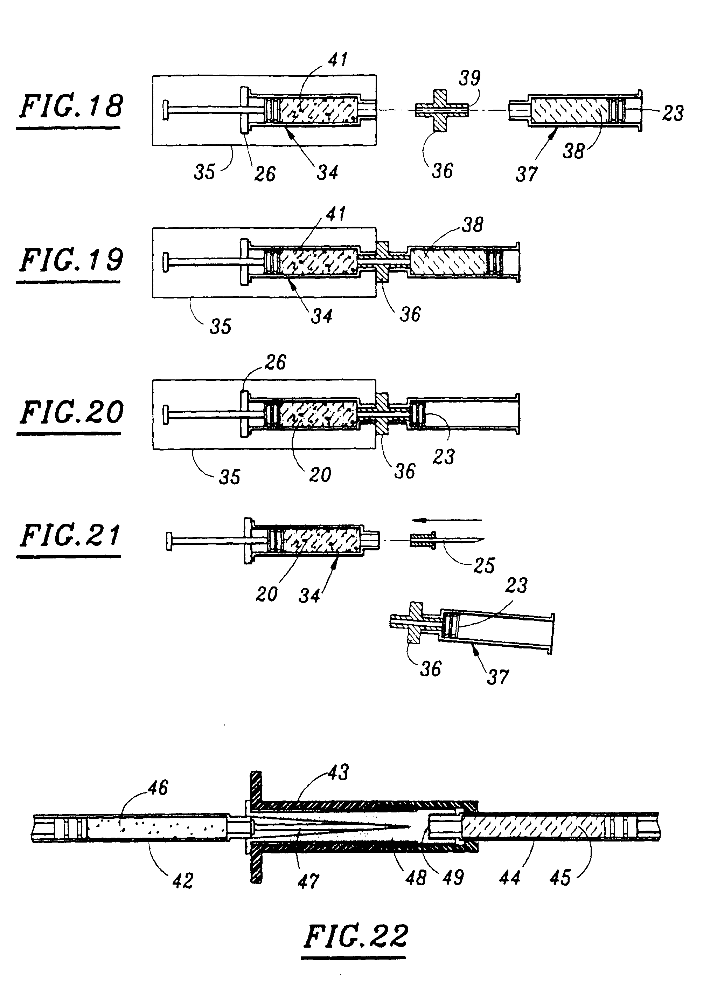

Dans la forme de réalisation des figures 18 à 21, la seringue 34 est conditionnée sous vide dans un emballage souple 35 et fixée à un connecteur 36 de liaison avec le réservoir 37 de liquide constitué par une carpule. Le connecteur 36 sert à percer l'emballage plastique 35 (figure 19) et à fixer la seringue 34 sur le réservoir 37 de liquide (eau).

Le connecteur 36 est percé d'un canal axial 39, de sorte que le percement de l'emballage plastique 35 par le connecteur 36 met en communication le volume de liquide 38 avec la forme sèche solide 41, ce qui aspire dans celle-ci, via le connecteur 36, le liquide 38 (figure 20), le piston 23 accompagnant le déplacement du liquide 38. Le réservoir 37 se vide donc automatiquement pour réhydrater le solide 41. Il est ensuite déconnecté de la seringue 34 (figure 21) et l'aiguille d'injection 25 est mise en place sur la seringue 34.

Dans le mode de réalisation de la figure 22, le dispositif comprend une seringue 42 reliée par un capuchon 43 à une carpule-septum 44 contenant le liquide 45. La seringue 42 comprenant le solide (forme sèche 46) est munie d'une aiguille d'injection 47 introduite et maintenue en place à l'intérieur du capuchon 43 dans une garniture 48 en un matériau souple tel qu'un élastomère, qui maintient l'aiguille 47 en place à l'intérieur du capuchon 43, en regard du septum 49. L'aiguille 47 est ainsi prête à être enfoncée dans le septum 49 pour provoquer le passage du liquide 45 dans la seringue 42 et ainsi la réhydratation du solide de principe actif 46.

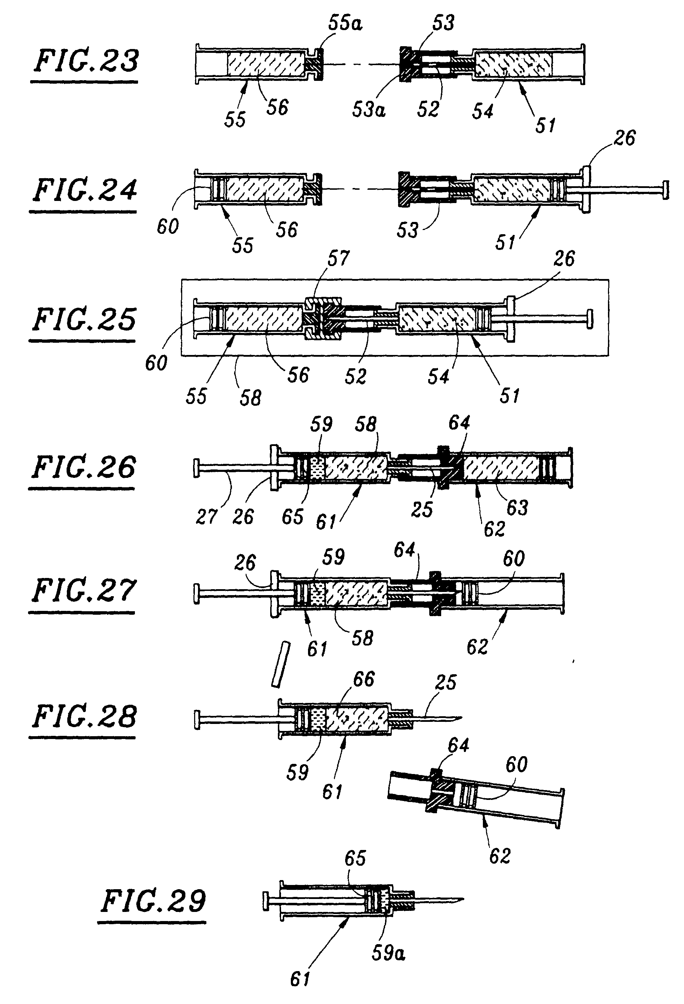

Dans le mode de réalisation du dispositif illustré aux figures 23 à 25, la seringue 51 est pourvue d'une aiguille d'injection 52 engagée dans un capuchon 53 à l'intérieur duquel elle peut coulisser pour mettre en communication le volume intérieur de là seringue 51 contenant le solide 54, avec le réservoir ou carpule 55 contenant le liquide 56. Ces deux éléments sont préparés indépendamment et associés ensuite dans le dispositif de réhydratation extemporanée (figures 24 et 25) par solidarisation du capuchon 53 avec l'extrémité 55a de la carpule 55 au moyen d'un raccord adapté 57 (figure 25).

L'ensemble est conditionné sous vide dans un emballage souple 58, prêt à l'emploi après extraction de cet emballage 58, enfoncement de l'aiguille 52 dans l'extrémité 55a et aspiration du liquide 56 dans le solide 54.

L'aiguille 52 est enfoncée dans le septum 53a qu'elle perce complètement au moment de la réhydratation sous vide du solide 54 par le liquide 56.

Dans le dispositif illustré aux figures 26 à 29, le procédé visé par l'invention prévoit d'ajouter à la forme sèche 58 une couche d'excipient 59 qu'on utilise à la suite de la préparation injectable comme piston liquide pour pousser les autres couches et réduire les pertes de principe actif lors de l'injection.

Le dispositif comprend, outre la seringue 61 contenant le principe actif solide 58, un réservoir 62 contenant un piston 60 et le liquide 63 de réhydratation, et un capuchon-septum 64 obturant le réservoir 62 du côté de la seringue 61 et dans lequel est engagée l'aiguille d'injection 25. La seringue 61 est munie de la pièce 26 de retenue de son piston 65 et de sa tige 27, la pièce 26 étant en appui contre l'extrémité du corps de la seringue.

Selon le procédé, après congélation du liquide contenant le principe actif et avant la lyophilisation ou séchage, on ajoute une quantité déterminée de solution d'excipient tel que du mannitol, en surface du liquide congelé. Ce volume est à son tour congelé et l'ensemble (58, 59) est ensuite lyophilisé. On obtient ainsi, entre le piston 65 et le solide 58 de principe actif sous vide, un volume 59 de lyophilisat ne contenant que l'excipient (mannitol). Ce volume 59 va servir, après réhydratation automatique et statique par percement du septum 64 par l'aiguille 25 (figure 27), et séparation du réservoir 62 à pousser la forme liquide 66 de principe actif. En fin d'injection, le volume 59 occupe les volumes morts 59a (figure 29) au fond de la seringue 61 et de l'aiguille 25.

Grâce au piston liquide 59, on parvient ainsi à éviter pratiquement toute perte de principe actif, ce qui constitue un avantage important en raison du coût de celui-ci.

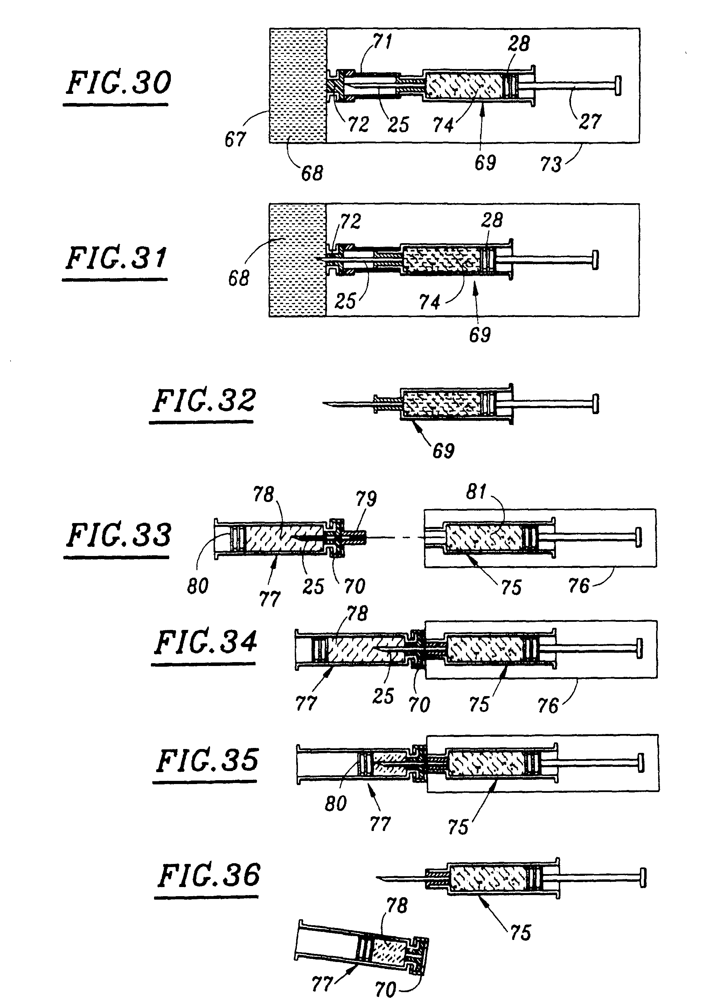

La forme de réalisation du dispositif illustré aux figures 30, 31, comprend un réservoir de liquide constitué par une poche souple 67 contenant le volume liquide 68 de réhydratation. La poche 67 est reliée à une seringue 69 par un bouchon 71 muni d'un septum 72 prêt à être percé par l'aiguille 25. La seringue 69 contenant la formulation solide 74 est conditionnée sous vide dans une enveloppe souple 73. L'aiguille 25 permet, par pression sur le piston 28, de connecter la formulation sous vide 74 et son volume liquide 68 de réhydratation (figure 31).

Une fois le mélange effectué, l'enveloppe de conditionnement 73 est enlevée, la poche 67 est séparée et le bouchon-septum 71 enlevé, la seringue 69 étant alors prête à l'emploi (figure 32).

Dans la réalisation illustrée aux figures 33 à 36, le dispositif comprend une seringue 75 conditionnée dans un emballage sous vide 76, et un réservoir 77 de liquide 78 muni d'un bouchon 70. L'aiguille d'injection 25 formant connecteur par son support 79 est préalablement introduite dans la carpule-réservoir 77 à travers le bouchon 70. Le réservoir 77 peut être relié à la seringue 75 par percement de l'emballage 76 par le raccord 79 (figure 34).

Une fois cette opération effectuée, le volume de liquide 78 et la formulation solide 81 sont mis en communication, de sorte que le liquide est aspiré dans la seringue 75 (figure 35). Après quoi il suffit d'enlever la carpule 77, son bouchon 70, ainsi que l'emballage plastique 76 pour rendre la seringue 75 prête à l'emploi (figure 36). Dans ce mode de réalisation l'aiguille 25 et son support 79 forment le connecteur proprement dit, l'aiguille 25 étant préalablement introduite dans le bouchon 70 de la carpule 77.

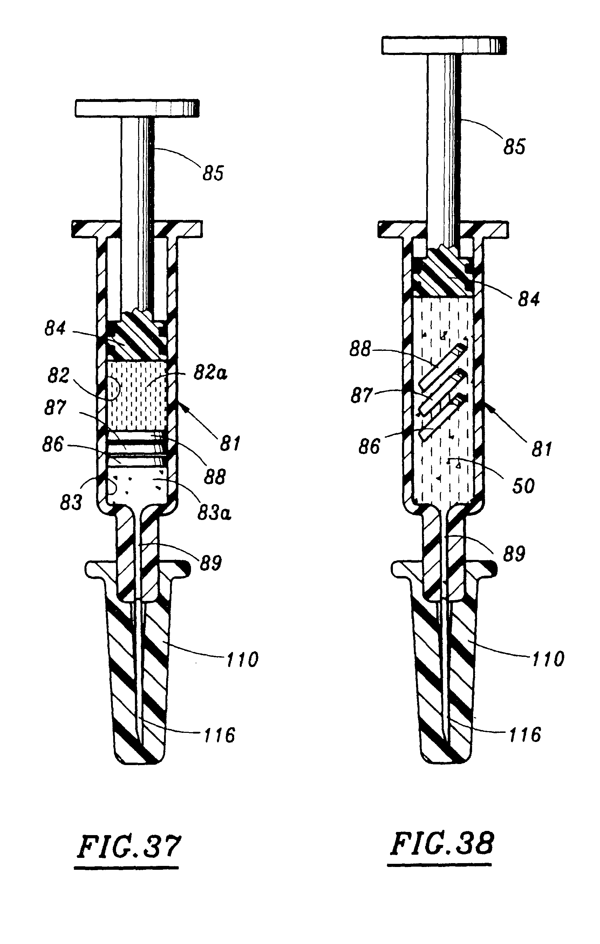

Dans le mode de réalisation représenté aux figures 37 à 39, le dispositif de conditionnement et de réhydratation sous vide de la préparation injectable comprend une seringue 81 avec une aiguille 116 enveloppée d'un bouchon 110. Cette seringue comporte deux compartiments 82, 83 contenant respectivement le liquide 82a et la formulation solide 83a. Ces compartiments sont délimités par un premier piston 84 solidaire d'une tige d'actionnement 85 et par trois autres pistons indépendants 86, 87, 88 juxtaposés entre le piston 84 et l'orifice d'injection 89. Ces trois pistons 86-88 sont indépendants, c'est-à-dire non solidarisés ensemble.

La seringue 81 est chargée avec un liquide contenant le principe actif 83. La préparation est ensuite lyophilisée et le lyophilisat 83a est conditionné sous vide dans le fond de la seringue avec les trois pistons plats et indépendants 86, 87, 88. Le volume liquide 82a de réhydratation est ensuite ajouté dans la seringue 81 puis le piston 84 avec sa tige 85, positionnée derrière le liquide 82a comme pour une seringue bicompartimentale. Le piston 84 est en une gomme standard et non rigide.

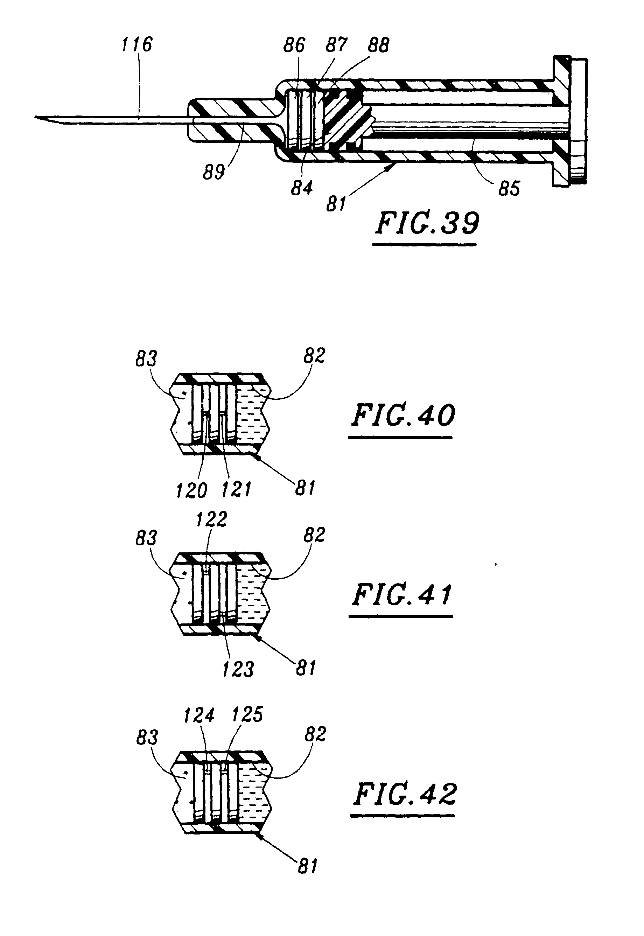

Lorsqu'on tire sur le piston 84 par sa tige 85, on aspire les trois pistons plats 86-88 (figure 38) qui basculent et font communiquer les formes solide 83a et liquide 82a. Durant cette aspiration et le coulissement du piston 84, les formes solide et liquide se mélangent et sont brassées par le mouvement des pistons 86-88. Le liquide (par exemple de l'eau), passe automatiquement dans le solide et reconstitue la préparation en liquide du principe actif, qui peut ensuite être immédiatement injectée (figure 39).

Ce système évite la seringue by-pass spécifique et peut être réalisé à partir d'une seringue standard.

Un positionnement des trois pistons indépendants 86-88 à plat dans la seringue pourrait empêcher un bon brassage du mélange liquide/lyophilisat mais l'arrangement des trois pistons évite ce risque. L'angle maximum de rotation des pistons est en relation avec la distance entre les pistons au repos. Ceux-ci, sont assez près les uns des autres pour éviter une rotation à 90° et dès que les deux chambres 82, 83 communiquent, les pistons 86-88 ne subissent plus de force susceptible de les déplacer jusqu'à ce qu'ils rentrent en contact avec le piston d'injection 84.

Toutefois, pour plus de sécurité, on peut en variante prévoir entre les pistons 86-88 des liens souples qui les réunissent deux à deux. Ces liens peuvent être centrés, tels que les liens 120 et 121 (Fig.40), ou bien asymétriques : liens 122, 123 (Fig.41 ) ou bien encore situés du même côté de l'axe de la seringue 81 : liens 124, 125 (Fig.42).

De tels agencements permettent de solidariser les pistons par un lien flexible, tout en laissant chaque piston libre de pivoter.

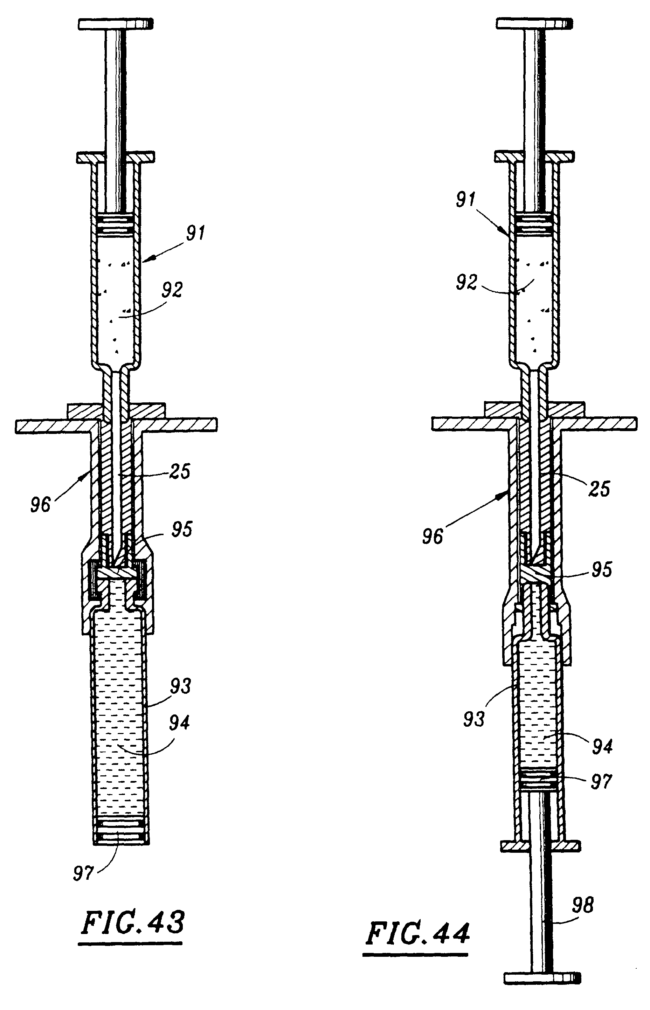

Le dispositif représenté à la figure 43 comporte une seringue 91 pré-remplie de solide sous vide 92, une carpule 93 contenant le liquide 94 et munie d'un septum 95, et un connecteur 96 de liaison entre la seringue 91 et le réservoir 93. L'aiguille d'injection 25 est introduite dans le connecteur 96, prête à perforer le septum 95.

Dès l'enfoncement du biseau terminal de l'aiguille 25 dans le septum 95, la forme solide 92 et la forme liquide 94 sont mises en communication et le liquide 94 est aspiré dans la formulation sèche 92 de la seringue 91.

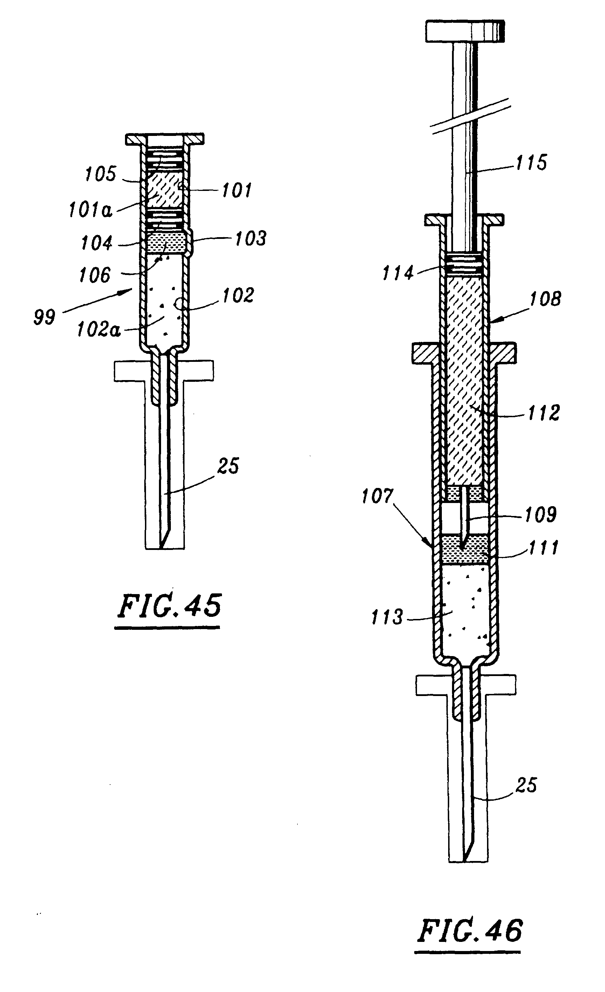

Dans la variante de réalisation de la figure 43, représentée à la figure 44, le piston 97 du réservoir de liquide 93 est équipé d'une tige 98 après que le liquide 94 ait été introduit dans ce réservoir 93, ce qui présente l'avantage d'éviter tout risque de fausse manoeuvre.

Dans le mode de réalisation de la figure 45, le dispositif comprend une seringue 99 du type à deux compartiments 101, 102 séparés par un by-pass central 103 obtenu par un décrochement latéral local de la paroi de la seringue entraînant une augmentation de la section à cet endroit. L'un des deux compartiments, à savoir le compartiment 101 devant contenir le liquide 101a de réhydratation, contient deux pistons indépendants 104, 105 entre lesquels peut être disposé le liquide.

Le procédé de mise en oeuvre de la préparation injectable au moyen de ce dispositif est le suivant.

Dans le compartiment 102 compris entre le by-pass 103 et l'aiguille d'injection 25 on charge et on congèle un liquide contenant le principe actif 102a, on ajoute au niveau du by-pass 103 une solution d'excipient 106 qui est ensuite congelée à son tour. L'excipient peut être une solution de mannitol diluée froide. On lyophilise l'ensemble sous vide, on dispose le premier piston 104 sur l'excipient sous vide 106, on remplit le second compartiment 101 de liquide 101 a, on place le second piston 105 sur le liquide 101a, on installe la tige (non représentée) du second piston 105).

On exerce ensuite par ce second piston 105 une pression qui écrase l'excipient lyophilisé sous vide 106, de sorte que le premier piston 104 coulisse et atteint le niveau du by-pass 103 ; le liquide 101a passe alors automatiquement via le by-pass 103 dans le premier compartiment 102 et réhydrate le solide sous vide 102a, et on obtient finalement la préparation prête à l'injection.

L'avantage du système par rapport à l'utilisation habituelle des by-pass est d'éviter les volumes non chargés de remise en solution ou suspension, et de permettre de remplir les volumes perdus au niveau du by-pass, des pistons du fond de la seringue et de l'aiguille avec une préparation liquide sans principe actif, à condition que le principe actif réhydraté ne se mélange pas avec l'excipient réhydraté, avant injection. C'est par exemple le cas des microsphères de PLGA (polylactic-glycic-acides).

On peut ainsi charger par exemple une quantité de 2ml et plus dans une seringue by-pass 99 initialement prévue pour 1ml seulement.

Le dispositif illustré à la figure 46 comporte une seringue 107 équipée d'une aiguille d'injection 25 et contenant une carpule-septum 108. Cette dernière est munie d'une courte aiguille 109 obturée par enfoncement dans un piston 111 d'injection de la seringue 107. La carpule-septum 108 peut contenir le liquide 112 de réhydratation et la seringue 107 peut contenir la forme sèche 113.

La carpule-septum 108 est équipée d'un piston 114 et d'une tige 115 de longueur suffisante pour pouvoir utiliser la carpule-septum 108 comme tige du piston d'injection 111. La carpute-septum 108, qui est l'équivalent d'une petite seringue avec sa courte aiguille 109, est pré-remplie de liquide 112 et positionnée dans le cylindre de la seringue 107. La courte aiguille 109 est obturée par enfoncement dans le piston d'injection 111. L'activation du dispositif est obtenue par enfoncement de l'aiguille 109 de la carpule-septum 108 dans le piston d'injection 111. Cet enfoncement permet de faire passer le liquide 112 dans le réservoir de la seringue 107 compris entre le piston d'injection 111 et l'aiguille 25 et qui contient le solide sous vide 113, ce qui aboutit à la reconstitution de la préparation injectable.

Dans ce type de réalisation, le piston d'injection 111 joue le rôle de septum ou de barrière entre le volume de liquide 112 et le solide sous vide 113. Ainsi, l'ensemble du dispositif est dans la même seringue 107, et l'aiguille 25 n'est pas utilisée pour percer le septum et pour servir d'élément de connexion.

Pour obtenir la forme sèche, dans le cas d'un semi-solide la dispersion liquide est chargée dans la seringue 107, lyophilisée ou séchée et conditionnée sous vide avec le piston d'injection 111.

Le produit obtenu par la mise en oeuvre du dispositif et du procédé qui viennent d'être décrits dans les différentes formes de réalisation illustrées aux dessins comprend, d'une manière générale, une forme sèche pour administration parentérale et conditionnée sous vide à l'intérieur d'un dispositif d'injection contenant également un volume liquide, prêt à être mélangé par aspiration avec la forme sèche pour reconstituer la préparation injectable.

La forme sèche peut être une forme lyophilisée ou une poudre obtenue après élimination d'un solvant.

La forme sèche peut contenir le seul principe actif, ou le principe actif et un excipient injectable, par exemple du mannitol.

Le volume contenant la forme sèche sous vide est égal au volume occupé par la préparation injectable obtenue après mélange de la forme sèche avec le liquide nécessaire.

Le liquide peut être de l'eau, ou un milieu aqueux ou un solvant organique avec ou sans eau, ou un liquide anhydre ou de l'huile injectable.

La préparation injectable obtenue peut être une solution liquide, ou une suspension solide dans un liquide , ou un gel ou encore une dispersion semi-solide.

Le vide nécessaire au procédé de préparation selon l'invention est un vide suffisant pour entraîner un appel du liquide de reconditionnement avant injection (hydratation ou autre) dans l'ensemble du volume à injecter sans laisser de bulle d'air, de zone vide ou de zone de produit encore sec.

Selon les procédés traditionnels de conditionnement des injectables secs ou lyophilisés, on peut utiliser un "vide partiel" d'air ou de gaz inerte (nitrogène, azote) avant de fermer le réservoir contenant la forme sèche pour éviter une surpression après bouchage.

Ce vide partiel peut être compensé par le bouchage avec un retour à la pression atmosphérique ou bien, une légère dépression peut-être maintenue dans le flacon ou dans la seringue pour éviter une surpression lors de l'addition du milieu liquide.

Il est bien entendu possible de boucher un flacon sous "vide total" après lyophilisation, mais cela ne présente aucun intérêt pour reconditionner le solide en dehors du cas précis de l'invention où le solide occupe tout le volume sous vide, et où le liquide vient exactement occuper ce volume vide directement dans le dispositif d'injection (seringue).

Le vide partiel peut représenter entre 0,9 et 0,6 atmosphère. Le vide total pourra être défini comme le vide correspondant à moins de 1/2 atmosphère et avantageusement aux basses pressions de 1/10 atmosphère et moins.

Ce vide total peut être également défini comme le vide obtenu par une pompe à vide utilisée par exemple pour un lyophilisateur. Une pompe rotative à vanne à doubles paliers peut atteindre 1.10⁻³ mbar ou 1µbar.

Le vide utilisé pour l'invention pourra donc être inférieur à 100 mbar ou avantageusement inférieur à 10 mbar ou encore inférieur à 0,1 mbar.

## Revendications

1. Procédé pour constituer une préparation injectable, dans lequel on prépare un volume de forme sèche d'un principe actif dans un volume déterminé d'un dispositif d'injection, lequel forme un moyen de conditionnement sous vide, et on introduit un volume d'un liquide dans ledit volume déterminé dudit dispositif pour former ladite préparation injectable par réhydratation extemporanée, **caractérisé en ce que** ledit volume de forme sèche de principe actif remplit complètement ledit volume déterminé, et y est maintenu sous vide, et **en ce que** l'on introduit un volume prédéterminé dudit liquide dans ledit volume déterminé, par aspiration grâce à l'action du vide pour que ledit volume déterminé se trouve complètement rempli par la formulation injectable.

2. Procédé selon la revendication 1, **caractérisé en ce que** le vide, dans ledit volume déterminé, est un vide total.

3. Procédé selon la revendication 2, dans lequel le vide total est un vide inférieur à 100 mbars.

4. Procédé selon la revendication 2, dans lequel ledit vide total est un vide inférieur à 10 mbars et avantageusement inférieur à 0,1 mbar.

5. Procédé selon la revendication 1, dans lequel on ajoute à la forme sèche une couche d'excipient (59) qu'on utilise à la suite de la préparation injectable comme un piston liquide pour pousser les autres couches (58) et réduire les pertes de principe actif lors de l'injection.

6. Procédé selon la revendication 5, dans lequel la forme sèche (58) est conditionnée dans une seringue (61) fixée à un dispositif automatique (62, 64) de réhydratation, **caractérisé en ce que** pour préparer la forme sèche, on congèle un liquide contenant le principe actif, on ajoute en surface du liquide congelé une quantité déterminée de solution d'excipient (59), on congèle cette solution d'excipient, on lyophilise l'ensemble, de sorte qu'on obtient ainsi, entre le piston (65) de la seringue et le solide (58) de principe actif sous vide, un volume (59) de lyophilisat ne contenant que l'excipient qui, après réhydratation automatique et déplacement du piston pour vider la seringue, occupe en fin d'injection un volume mort au fond de la. seringue et de l'aiguille d'injection (25) .

7. Procédé selon l'une des revendications 1 à 6, dans lequel on prépare la forme sèche dans une seringue (99) du type à by-pass constitué par un accroissement (103) de section dans une zone médiane de la seringue, **caractérisé en ce qu'**on charge et congèle dans un compartiment (102) compris entre le by-pass et une aiguille d'injection, un liquide (101a) contenant le principe actif, on ajoute au niveau du by-pass une solution d'excipient (106) qui est ensuite congelée à son tour, on lyophilise l'ensemble sous vide, on dispose un premier piston (104) sur l'excipient sous vide, on remplit le second compartiment (101) de liquide (101a), on place un second piston (105) sur le liquide, on installe la tige du second piston (105), on exerce par ce second piston une pression qui écrase l'excipient lyophilisé, de sorte que, lorsque le premier piston atteint le by-pass, le liquide passe automatiquement via le by-pass dans le premier compartiment et réhydrate le solide sous vide, et qu'on obtient finalement une préparation prête à l'injection (Fig. 42).

8. Procédé selon la revendication 1, **caractérisé en ce que** la forme sèche (3) étant constituée de principe actif avec ou sans excipient injectable, on prépare la forme sèche après avoir réparti la forme sèche obtenue en différents volumes précis dans des dispositifs (11), qu'on referme ensuite sous vide (Fig. 1-7).

9. Procédé selon la revendication 8, **caractérisé en ce qu'**on mélange le liquide et la forme sèche par va-et-vient entre deux seringues (1, 6) à travers un connecteur (Fig. 6).

10. Dispositif d'injection pour la mise en oeuvre du procédé de constitution d'une préparation injectable selon l'une quelconque des revendications 1 à 9, comprenant des moyens de conditionnement sous vide (19) d'une forme sèche (18) remplissant complètement un volume déterminé du dispositif d'injection, des moyens de conditionnement (21) d'un volume prédéterminé de liquide (22) de réhydratation extemporanée et des moyens de connexion (29) entre lesdits moyens de conditionnement afin d'ajouter par aspiration le liquide à la forme sèche, pour remplir la totalité dudit volume déterminé, les moyens de conditionnement sous vide (19) constituant le dispositif d'injection après ladite réhydratation extemporanée.

11. Dispositif selon la revendication 10, **caractérisé en ce que** les moyens de conditionnement sous vide d'une forme sèche sont une seringue (11, 12, 19) étanche aux gaz, et lesdits moyens de conditionnement de liquide sont un réservoir (16) contenant un piston (10) .

12. Dispositif selon la revendication 10, **caractérisé en ce que** les moyens de connexion comprennent soit un septum (29) et une aiguille d'injection (25), soit un robinet (2) (Fig, 10-11).

13. Dispositif selon la revendication 10, **caractérisé en ce que** la seringue est conditionnée sous vide dans un emballage souple (8, 31) et fixée à un connecteur de liaison avec le réservoir de liquide (16).

14. Dispositif selon la revendication 10, **caractérisé en ce que** la seringue (19) est pourvue d'une aiguille d'injection (25) engagée dans un capuchon (29) à l'intérieur duquel elle peut coulisser pour mettre en communication la seringue avec le réservoir de liquide (21), et **en ce que** l'ensemble est conditionné dans un emballage sous vide (3) (Fig. 11).

15. Dispositif selon la revendication 10, **caractérisé en ce que** le réservoir de liquide est une poche souple (67) reliée par un bouchon-septum (71) à la seringue (69), cette dernière étant conditionnée sous vide (Fig. 30-32).

16. Dispositif selon la revendication 11, **caractérisé en ce que** la seringue (75) est conditionnée dans un emballage sous vide (76) et pourvue d'une aiguille d'injection (25) formant connecteur, introduite dans le réservoir de liquide à travers un bouchon (70) et reliée à la seringue à travers l'emballage (76) (Fig. 33-36).

17. Dispositif selon la revendication 11, **caractérisé en ce que** la seringue (81) comporte deux compartiments (82, 83) délimités par un premier piston (84) solidaire d'une tige d'actionnement (85) et par des pistons indépendants, par exemple trois (86, 87, 88) (Fig. 37-39), éventuellement liés entre eux.

18. Dispositif selon la revendication 11, **caractérisé en ce que** la seringue (99) est du type à deux compartiments (101, 102) séparés par un by-pass (103) central, et **en ce que** l'un des deux compartiments, destiné à contenir le liquide (101a) de réhydratation, contient deux pistons indépendants (104, 105) entre lesquels peut être disposé le liquide (Fig. 45).

19. Dispositif selon la revendication 11, **caractérisé en ce que** la seringue (107) contient une carpule-septum (108) pourvue d'une aiguille (109) obturée par enfoncement dans un piston (111) d'injection de la seringue, la carpule-septum pouvant contenir le liquide de réhydratation (112) et la seringue pouvant contenir la forme sèche (113) (Fig. 46).

20. Dispositif selon la revendication 11, **caractérisé en ce que** la carpule-septum est équipée d'un piston (114) et d'une tige (115) de longueur suffisante pour pouvoir utiliser la carpule-septum comme tige du piston d'injection (111), après enfoncement de l'aiguille (109) de la carpule-septum dans le piston d'injection afin de faire passer le liquide contenu dans la carpule-septum dans le réservoir de la seringue compris entre le piston d'injection et l'aiguille d'injection et contenant un solide sous vide, ce qui permet de reconstituer la préparation injectable (Fig, 46) .

21. Dispositif selon la revendication 11, **caractérisé en ce que** la seringue (42) est reliée par un capuchon (43) à une carpule-septum (44) contenant le liquide (45), la seringue étant munie d'une aiguille d'injection (47) introduite et maintenue en place à l'intérieur du capuchon, en regard du septum (49) de la carpule-septum, prête à être enfoncée dans le septum pour provoquer la réhydratation du solide (46) de principe actif contenu dans la seringue (Fig. 22).

## Patentansprüche

1. Verfahren für die Anfertigung eines injizierbaren Präparates, bei dem ein Volumen einer Trockenmasse eines aktiven Prinzips in einem berechneten Volumen eines Injektionsgerätes zubereitet wird, das ein Mittel für die Vakuumverpackung bildet, und ein Volumen einer Flüssigkeit in das berechnete Volumen der Vorrichtung eingefüllt wird, um das injizierbare Präparat durch unmittelbare Rehydratisation herzustellen, **dadurch gekennzeichnet, dass** das Volumen der Trockenmasse des aktiven Prinzips das berechnete Volumen vollkommen ausfüllt und dort unter Vakuum gehalten wird, und dadurch, dass ein bestimmtes Volumen der Flüssigkeit in das berechnete Volumen unter aufgrund der Wirkung des Vakuums durch Ansaugung so eingefüllt wird, dass das berechnete Volumen vollkommen mit der injizierbaren Rezeptur ausgefüllt wird.

2. Verfahren nach Anspruch 1, **dadurch gekennzeichnet, dass** das Vakuum in dem berechneten Volumen ein vollständiges Vakuum ist.

3. Verfahren nach Anspruch 2, **dadurch gekennzeichnet, dass** das vollständige Vakuum ein Vakuum unter 100 mbar ist.

4. Verfahren nach Anspruch 2, **dadurch gekennzeichnet, dass** das vollständige Vakuum ein Vakuum unter 10 mbar und vorzugsweise ein Vakuum unter 0,1 mbar ist.

5. Verfahren nach Anspruch 1, **dadurch gekennzeichnet, dass** der Trockenmasse eine Schicht einer Trägermasse (59) beigemischt wird, die im Anschluß an die Herstellung des injizierbaren Präparats als flüssiger Kolben verwendet wird, um die anderen Schichten (58) zu komprimieren und den Verlust des aktiven Prinzips während der Injektion zu reduzieren.

6. Verfahren nach Anspruch 5, bei dem die Trockenmasse (58) in eine Spritze (61) eingefüllt wird, die an einer automatischen Vorrichtung (62, 64) für die Rehydratisation befestigt ist, **dadurch gekennzeichnet, dass** für die Zubereitung der Trockenmasse eine Flüssigkeit, welche das aktive Prinzip enthält, eingefroren wird und auf die Oberfläche der eingefrorenen Flüssigkeit eine bestimmte Menge einer Trägerlösung (59) aufgetragen und diese Trägerlösung dann eingefroren wird und die gesamte Masse so gelöst wird, dass zwischen dem Kolben (65) der Spritze und der Trockenmasse (58) des unter Vakuum stehenden aktiven Prinzips ein Volumen (59) der Lösung erhält, die nur . die Trägermasse enthält, und die nach der automatischen Rehydratisation und der Verschiebung des Kolbens für die Entleerung der Spritze ein Totvolumen am Boden der Spritze und der Injektionsnadel (25) bildet.

7. Verfahren nach einem der Ansprüche 1 bis 6, bei dem die Trockenmasse in einer Spritze (99) von der Art eines Bypass zubereitet wird, der aus einer Vergrößerung (103) des Querschnitts im mittleren Bereich der Spritze besteht, **dadurch gekennzeichnet, dass** in ein Abteil (102), das zwischen dem Bypass und einer Injektionsnadel liegt, eine Flüssigkeit (101a) eingefüllt und eingefroren wird, welche das aktive Prinzip enthält, und an dem Bypass eine Trägerlösung (106) zugegeben wird, die dann ebenfalls eingefroren wird, und anschließend die Gesamtmasse unter Vakuum gelöst wird, und dann ein erster Kolben (104) auf die unter Vakuum stehende Trägerlösung aufgesetzt wird, und dann das zweite Abteil (101) mit einer Flüssigkeit (101a) befüllt wird, und dann ein zweiter Kolben (105) auf die Flüssigkeit aufgesetzt wird, und die Stange des zweiten Kolbens (105) installiert wird, und mit Hilfe dieses zweiten Kolbens ein Druck ausgeübt wird, welcher die gelöste Trägermasse so zusammenpresst, dass wenn der erste Kolben den Bypass erreicht, die Flüssigkeit automatisch durch den Bypass in das erste Abteil fließt und die unter Vakuum stehende Trockenmasse rehydratisiert, so dass man schließlich ein Präparat erhält, das für eine Injektion bereit ist (siehe Fig. 42).

8. Verfahren nach Anspruch 1, **dadurch gekennzeichnet, dass** die Trockenmasse (3), die aus dem aktiven Prinzip mit oder ohne injizierbare Trägermasse besteht, die Trockenmasse hergestellt wird, nachdem die in verschiedenen genauen Volumen in den Vorrichtungen (11) hergestellte Trockenmasse verteilt worden ist, die anschließend wieder unter Vakuum verschlossen werden (siehe die Fig. 1 bis 7).

9. Verfahren nach Anspruch 8, **dadurch gekennzeichnet, dass** das Gemisch aus der Flüssigkeit und der Trockenmasse über einen Anschluss durch Hubbewegung zwischen zwei Spritzen (1, 6) vermischt wird (siehe Fig. 6).

10. Injektionsgerät für die Durchführung des Verfahrens für die Anfertigung eines injizierbaren Präparats nach einem der Ansprüche 1 bis 9, welches Mittel (19) für die Verpackung unter Vakuum einer Trockenmasse (18) enthält, die ein bestimmtes Volumen der Injektionsvorrichtung vollständig ausfüllt, und Mittel (21) für die Verpackung eines bestimmten Volumens einer Flüssigkeit (22) für die unmittelbare Rehydratisation und Mittel (29) für die Verbindung zwischen den Mitteln für die Verpackung enthält, um durch Ansaugung die Flüssigkeit mit der Trockenmasse zu vermischen, um das gesamte berechnete Volumen auszufüllen, wobei die Mittel (19) für die Verpackung unter Vakuum das Injektionsgerät nach der unmittelbaren Rehydratisation bildet.

11. Injektionsgerät nach Anspruch 10, **dadurch gekennzeichnet, dass** die Mittel für die Verpackung unter Vakuum der Trockenmasse aus einer luftdichten Spritze (11, 12, 19) bestehen und die Mittel für die Verpackung der Flüssigkeit aus einem Behälter (16) bestehen, der einen Kolben (10) enthält.

12. Injektionsgerät Verfahren nach Anspruch 10, **dadurch gekennzeichnet, dass** die Verbindungsmittel entweder eine Trennwand (29) und eine Injektionsnadel (25) oder aber ein Ventil (2) enthalten (siehe Fig. 10 bis 11).

13. Injektionsgerät nach Anspruch 10, **dadurch gekennzeichnet, dass** die Spritze unter Vakuum in einer weichen Verpackung (8, 31) verpackt wird und an einer Verbindung mit dem Flüssigkeitsbehälter (16) befestigt ist.

14. Injektionsgerät nach Anspruch 10, **dadurch gekennzeichnet, dass** die Spritze (19) mit einer Injektionsnadel (25) ausgerüstet ist, die in einen Deckel (29) eingesetzt ist, in dessen Innenseite sie gleiten kann, um die Spritze an den Flüssigkeitsbehälter (21) anzuschließen, und dadurch, dass die Gesamtmasse in einer Vakuumverpackung (3) verpackt ist (siehe Fig. 11).

15. Injektionsgerät nach Anspruch 10, **dadurch gekennzeichnet, dass** der Flüssigkeitsbehälter aus einem weichen Beutel (67) besteht, welcher mit Hilfe eines Stöpsels (71) mit der Spritze (69) verbunden ist, die unter Vakuum verpackt worden ist (siehe Fig. 30 bis 32).

16. Injektionsgerät nach Anspruch 11, **dadurch gekennzeichnet, dass** die Spritze (75) in einer Vakuumverpackung (76) verpackt und mit einer Injektionsnadel (25) ausgestattet ist, welche einen Anschluß bildet, und die über einen Stöpsel (70) in den Flüssigkeitsbehälter eingeführt wird, und durch die Verpackung (76) mit der Spritze verbunden ist (siehe Fig. 33 bis 36).

17. Injektionsgerät nach Anspruch 11, **dadurch gekennzeichnet, dass** die Spritze (81) zwei Abteile (82, 83) enthält, welche durch einen ersten Kolben (84), der mit einer Betätigungsstange (85) verbunden ist, oder aber zum Beispiel mit Hilfe von drei unabhängigen Kolben (86, 87, 88) gebildet werden, die eventuell miteinander verbunden sind (siehe Fig. 37 bis 39).

18. Injektionsgerät nach Anspruch 11, **dadurch gekennzeichnet, dass** die Spritze (99) aus einer Spritze mit zwei Abteilen (101, 102) besteht, welche durch einen zentralen Bypass (103) voneinander getrennt sind, und dadurch, dass eines der beiden Abteile, welches die Flüssigkeit (101a) für die Rehydratisation enthält, zwei unabhängige Kolben (104, 105) enthält, zwischen denen die Flüssigkeit eingefüllt werden kann (siehe Fig. 45).

19. Injektionsgerät nach Anspruch 11, **dadurch gekennzeichnet, dass** die Spritze (107) eine Trennkapsel (108) enthält, die mit einer Nadel ausgestattet (109) ist, welche durch das Einpressen in einen Kolben (111) der Injektionsspritze verschlossen wird, wobei diese Trennkapsel die Flüssigkeit für die Rehydratisation (112) enthalten kann, während die Spritze die Trockenmasse (113) enthalten kann (siehe Fig. 46).

20. Injektionsgerät nach Anspruch 11, **dadurch gekennzeichnet, dass** die Trennkapsel mit einem Kolben (114) und einer Stange (115) mit einer Länge ausgestattet ist, die ausreicht, um die Trennkapsel als Stange für den Injektionskolben (111) zu verwenden, nachdem die Nadel (109) der Trennkapsel in den Injektionskolben gedrückt worden ist, um die Flüssigkeit, die in dieser Trennkapsel enthalten ist, in den Behälter der Spritze zu leiten, der zwischen dem Injektionskolben und der Injektionsnadel angeordnet ist, und eine unter Vakuum stehende Trockenmasse enthält, so dass es möglich ist, das injizierbare Präparat herzustellen (siehe Fig. 46).

21. Injektionsgerät nach Anspruch 11, **dadurch gekennzeichnet, dass** die Spritze (42) mit Hilfe einer Verschlusskappe (43) an eine Trennkapsel (44) angeschlossen ist, welche die Flüssigkeit (45) enthält, und die Spritze mit einer Injektionsnadel (47) ausgerüstet ist, die in das Innere der Verschlusskappe eingeführt und dort gegenüber der Trennwand (49) der Trennkapsel gehalten wird, und dann in die Trennwand gedrückt werden kann, um die Rehydratisation der Trockenmasse (46) des aktiven Prinzips einzuleiten, das in der Spritze enthalten ist (siehe Fig. 22).

## Claims

1. Method of forming an injectable preparation, in which a volume of an active ingredient in dry form is prepared in a specific volume of an injection device, which forms a vacuum packing means, and a volume of a liquid is. introduced into the said specific volume of the said device in order to form the said injectable preparation by extemporaneous rehydration, **characterised in that** the said volume of active ingredient in dry form completely fills the said specific volume and is kept there under a vacuum, and that a predetermined volume of the said liquid is introduced into the said specific volume by suction due to the action of the vacuum in order for the said specific volume to be completely filled by the injectable formulation.

2. Method as claimed in Claim 1, **characterised in that** the vacuum in the said specific volume is a total vacuum.

3. Method as claimed in Claim 2, in which the total vacuum is a vacuum less than 100 mbars.

4. Method as claimed in Claim 2, in which the said total vacuum is a vacuum less than 10 mbars and advantageously less than 0.1 mbar.

5. Method as claimed in Claim 1, in which there is added to the dry form a layer of excipient (59) which is used following the injectable preparation as a liquid piston in order to push the other layers (58) and to reduce the losses of active ingredient during injection.

6. Method as claimed in Claim 5, in which the dry form (58) is packaged in a syringe (61) fixed to an automatic rehydration device (62, 64), **characterised in that** in order to prepare the dry form a liquid containing the active ingredient is frozen, a specific quantity of excipient solution is added at the surface of the frozen liquid, this excipient solution is frozen, the combination is freeze-dried so that between the piston (65) of the syringe and the solid (58) active ingredient under vacuum a volume (59) of lyophilisate is obtained containing only the excipient which, after automatic rehydration and displacement of the piston in order to empty the syringe, at the end of the injection occupies a dead volume at the base of the syringe and of the injection needle (25).

7. Method as claimed in one of Claims 1 to 6, in which the dry form is prepared in a syringe (99) of the type having a bypass formed by an increase (103) in cross-section in a median zone of the syringe, **characterised in that** a liquid (101a) containing the active ingredient is introduced and frozen in a compartment (102) included between the bypass and an injection needle, a solution of excipient (106) is added at the level of the bypass and is then frozen in its turn, the whole combination is freeze-dried under a vacuum, a first piston (104) is disposed on the excipient under vacuum, the second compartment (101) is filled with liquid (101a), a second piston (105) is placed on the liquid, the rod of the second piston (105) is installed, a pressure is exerted by this second piston which squeezes the freeze-dried excipient such that when the first piston reaches the bypass the liquid passes automatically via the bypass into the first compartment and rehydrates the solid under vacuum, and finally a preparation is obtained which is ready for injection (Figure 42).

8. Method as claimed in Claim 1, **characterised in that** as the dry form (3) is constituted by active ingredient with or without injectable excipient, the dry form is prepared after having distributed the dry form obtained in different precise volumes in devices (11) which are then closed under a vacuum (Figures 1 to 7).

9. Method as claimed in Claim 8, **characterised in that** the liquid and the dry form are mixed by to-and-fro movement between two syringes (1, 6) through a connector (Figure 6).

10. Injection device for carrying out the method of forming an injectable preparation as claimed in any one of Claims 1 to 9, comprising means for vacuum packing ( 19) of a dry form which completely fills a specific volume of the injection device, means for packaging (21) of a predetermined volume of liquid (22) for extemporaneous rehydration and means for connection (29) between the said packaging means in order to add the liquid by suction to the dry form, to fill the totality of the said specific volume, the means for vacuum packing (19) constituting the injection device after the said extemporaneous rehydration.

11. Device as claimed in Claim 10, **characterised in that** the means for vacuum packing of a dry form are a gas-tight syringe (11, 12, 19), and the said means for packaging liquid are a reservoir (16) containing a piston (10).

12. Device as claimed in Claim 10, **characterised in that** the connection means comprise either a septum (29) and an injection needle (25) or a valve (2) (Figures 10 to 11).

13. Device as claimed in Claim 10, **characterised in that** the syringe is vacuum packed in a flexible packaging (8, 31) and fixed to a connector for connection to the reservoir of liquid (16).

14. Device as claimed in Claim 10, **characterised in that** the syringe (19) is provided with an injection needle (25) engaged in a cap (29) inside which it can slide in order to put the syringe into communication with the reservoir of liquid (21), and that the whole combination is packaged in a vacuum packaging (3) (Figure 11).

15. Device as claimed in Claim 10, **characterised in that** the reservoir of liquid is a flexible pocket (67) connected by a plug-septum (71) to the syringe (69), this latter being vacuum packed (Figures 30 to 32).

16. Device as claimed in Claim 11, **characterised in that** the syringe (75) is packaged in a vacuum packaging (76) and provided with an injection needle (25) forming a connector, introduced into the reservoir of liquid through a plug (70) and connected to the syringe through the packaging (76) (Figures 33 to 36).

17. Device as claimed in Claim 11, **characterised in that** the syringe (81) has two compartments (82, 83) delimited by a first piston (84) integral with an actuating rod (85) and by independent pistons, for example three such pistons (86, 87, 88) (Figures 37 to 39) which are optionally connected to one another.

18. Device as claimed in Claim 11, **characterised in that** the syringe (99) is of the type having two compartments (101, 102) separated by a central bypass (103), and that one of the two compartments, intended to contain the rehydration liquid (101a), contains two independent pistons between which the liquid can be disposed (Figure 45).

19. Device as claimed in Claim 11, **characterised in that** the syringe (107) contains a cartridge-septum (108) provided with a needle (109) closed off by driving into an injection piston (11) of the syringe, wherein the cartridge-septum can contain the rehydration liquid (112) and the syringe can contain the dry form (113) (Figure 46).

20. Device as claimed in Claim 11, **characterised in that** the cartridge-septum is equipped with a piston (114) and a rod (115) of sufficient length to be able to use the cartridge-septum as the rod of the injection piston (111), after driving the needle (109) of the cartridge-septum into the injection piston in order to cause the liquid contained in the cartridge-septum to pass into the reservoir of the syringe between the injection piston and the injection needle, and containing a solid under vacuum, which enables the injectable preparation to be reconstituted (Figure 46).

21. Device as claimed in Claim 11, **characterised in that** the syringe (42) is connected by a cap (43) to a cartridge-septum (44) containing the liquid (45), the syringe being provided with an injection needle (47) introduced and held in place inside the cap, facing the septum (49) of the cartridge-septum, ready to be driven into the septum in order to bring about the rehydration of the solid active ingredient (46) contained in the syringe (Figure 22).
